(19) Europäisches Patentamt

European Patent Office

Office européen des brevets

(11) **EP 0 959 911 B1**

(12) **FASCICULE DE BREVET EUROPEEN**

(45) Date de publication et mention
de la délivrance du brevet:
**01.12.2004 Bulletin 2004/49**

(51) Int Cl.⁷: **A61M 1/10**

(21) Numéro de dépôt: **97906219.7**

(22) Date de dépôt: **19.02.1997**

(86) Numéro de dépôt international:
**PCT/FR1997/000302**

(87) Numéro de publication internationale:
**WO 1997/030739 (28.08.1997 Gazette 1997/37)**

(54) **CIRCUIT DE PILOTAGE EN COMBINAISON AVEC UNE POMPE D'ASSISTANCE CARDIAQUE IMPLANTABLE DU TYPE A BALONNET DE CONTREPRESSION**

**STEUERSCHALTUNG MIT EINER IMPLANTIERBAREN HERZUNTERSTÜTZUNGSPUMPE IN FORM EINER GEGENDRUCKBALLONPUMPE**

**CONTROL CIRCUIT IN COMBINATION WITH AN IMPLANTABLE CARDIAC ASSISTANCE PUMP WITH A COUNTERPRESSURE BALLOON**

(84) Etats contractants désignés:
**AT BE CH DE DK ES FI FR GB GR IE IT LI LU MC NL PT SE**

(30) Priorité: **21.02.1996 FR 9602135**

(43) Date de publication de la demande:
**01.12.1999 Bulletin 1999/48**

(73) Titulaire: **Synthelabo Biomedical, S.A.**
**92350 Le Plessis-Robinson (FR)**

(72) Inventeur: **FRANCHI, Pierre**
**F-94400 Vitry-sur-Seine (FR)**

(74) Mandataire: **Dupuis-Latour, Dominique**
**SEP Pagenberg & Associés**
**14 boulevard Malesherbes**
**75008 Paris (FR)**

(56) Documents cités:
EP-A- 0 654 276          WO-A-93/08874
WO-A-95/28974            US-A- 4 051 841
US-A- 4 137 910          US-A- 5 098 370
US-A- 5 222 980

## Description

L'invention concerne un dispositif d'assistance cardiaque implantable du type à ballonnet de contrepression.

La technique du ballonnet intraaortique de contrepression est bien connue pour apporter une assistance hémodynamique efficace au ventricule gauche en cas d'insuffisance cardiaque congestive : le ballonnet, introduit dans la branche descendante de l'aorte, est gonflé pendant la phase diastolique du cycle cardiaque et injecte de ce fait un volume de sang supplémentaire dans le réseau artériel en amont et en aval de sa position. Dégonflé pendant la systole cardiaque consécutive, il diminue la charge du ventricule gauche et permet ainsi d'accroître le débit sanguin. Le bilan hémodynamique est positif : augmentation de la fraction d'éjection, diminution de la pression télédiastolique. Ainsi, le ballonnet délivre le complément d'énergie que le ventricule n'est plus en mesure de fournir, et l'état du patient est très sensiblement amélioré.

On a déjà proposé des systèmes implantés permettant de mettre en oeuvre cette technique de façon entièrement autonome, comme cela est par exemple décrit dans le US-A-5 222 980, ou encore dans le EP-A-0 876 168 (WO-A-97/26929), publié le 31 juillet 1997 et faisant partie de l'état de la technique seulement au titre de l'art. 54(3) CBE, et intitulé *Pompe d'assistance car diaque implantable du type à ballonnet de contrepression.*

Ces documents décrivent tous deux une pompe d'assistance cardiaque implantable permanente insérée dans l'aorte descendante, fonctionnant sur le principe précité du ballonnet de contrepression et constituée d'une membrane souple et élastique ayant la forme d'un manchon dont l'axe est confondu avec celui de l'aorte et disposé en lieu et place d'un tronçon d'aorte réséqué. La membrane est contenue dans une chambre rigide épousant sensiblement la forme de la membrane au repos, et dans laquelle l'injection d'un fluide hydraulique en provenance d'un générateur externe a pour effet de comprimer la membrane et donc de réduire le volume de sang qu'elle contient. À l'inverse, l'extraction du fluide provoque l'augmentation de son volume intérieur, donc le remplissage de la pompe.

Plus précisément, la présente invention a pour objet un circuit de commande d'une telle pompe d'assistance cardiaque implantable qui assure un pilotage du dispositif tel que le comportement de celui-ci soit le plus physiologique possible, c'est-à-dire qui pallie l'insuffisance cardiaque sans demander d'effort excessif ou anti-naturel au myocarde affaibli, et qui génère dans l'ensemble du système cardiovasculaire un débit sanguin présentant une caractéristique, notamment d'onde de pression, la plus proche possible de celle d'un organisme sain.

Un autre but de l'invention est de réaliser un pilotage évolutif de la pompe, adapté à la demande hémodynamique réelle du patient de manière à, d'une part, limiter la consommation énergétique du dispositif implanté et, d'autre part et surtout, perturber le moins possible l'équilibre homéostatique naturel et éviter ainsi d'éventuelles complications organiques à long terme suite à l'implantation de la pompe.

À cet effet, l'invention propose un dispositif d'assistance cardiaque implantable du type à ballonnet de contrepression du type décrit par le US-A-5 222 980 précité et comportant les éléments énoncés par la revendication 1. Les sous-revendications visent des formes de mise en oeuvre particulières avantageuses.

D'autres caractéristiques de l'invention apparaîtront à la lecture de la description ci-dessous d'un exemple de mise en oeuvre de l'invention.

La figure 1 est une vue schématique montrant la pompe de l'invention, son circuit de commande et l'environnement dans lequel est implanté l'ensemble du dispositif.
La figure 2 illustre une modélisation du fonctionnement de la pompe de la figure 1.
La figure 3 est une représentation de la caractéristique de l'onde de pression en fonction du temps sur un cycle cardiaque, avec et sans assistance par la pompe de l'invention.

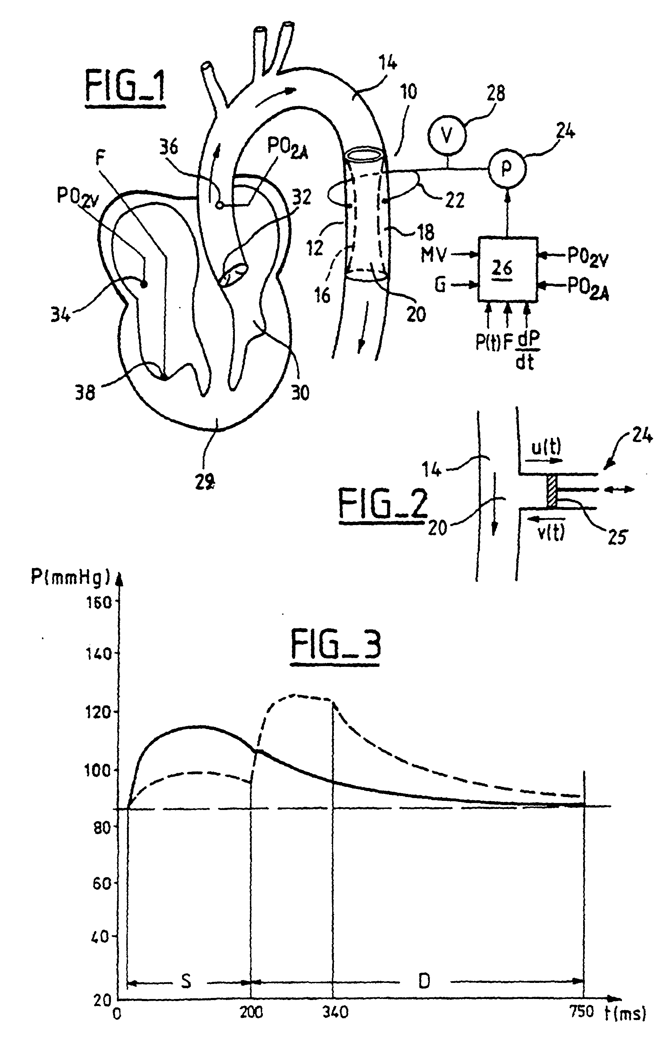

Sur la figure 1, on a représenté une pompe d'assistance cardiaque implantable, de type en elle-même connu (par exemple d'après le US-A-5 222 980 ou le WO-A-97/26929 précités), dont l'élément principal 10 comprend un corps rigide 12, typiquement en forme de cylindre de révolution, ouvert à ses deux extrémités et inséré dans l'aorte descendante 14, l'axe de l'aorte et l'axe du corps 12 étant confondus et ces deux éléments étant sensiblement de même diamètre.

Le corps rigide 12 contient une membrane souple 16. Dans la forme de réalisation illustrée, la membrane 16 a au repos une forme homologue de celle du corps 12, de manière à épouser sensiblement la forme de ce dernier, auquel elle est solidarisée aux deux extrémités sur toute sa périphérie.

On définit ainsi entre le corps 12 et la membrane 16 un espace intermédiaire 18, fermé, de volume variable et, à l'intérieur de la membrane 16, un espace central 20, également de volume variable, ce dernier volume diminuant lorsqu'augmente le volume 18, et inversement.

L'augmentation de volume de l'espace 18 résulte de l'injection d'un fluide hydraulique (typiquement une solution saline aqueuse biocompatible, par exemple un sérum physiologique) en un ou, de préférence, plusieurs points reliés par l'intermédiaire d'une conduite 22 à une source de pression variable 24 contrôlée par une électronique de commande 26. On peut avantageusement prévoir en outre un réservoir de fluide hydraulique 28 sous forme d'un septum accessible par voie percutanée au moyen d'une aiguille hypodermique pour permettre l'ajustement du volume et/ou de la salinité du fluide, ou le vidanger.

L'invention a plus particulièrement pour objet le circuit 26 qui pilote la source de pression 24.

La pompe peut être modélisée, comme illustré figure 2, sous la forme d'un piston 25 dont le déplacement, dans un sens ou dans l'autre, se traduit par une variation du volume aortique 20. Cette variation artificielle du volume 20 dans l'aorte 14 est comparable à la dilatation naturelle d'une artère saine résultant de l'élasticité propre des parois artérielles, et d'amplitude supérieure.

Plus précisément, pendant la systole cardiaque, la contraction du myocarde 29 provoque successivement la montée en pression du ventricule gauche 30, l'ouverture de la valvule aortique 32 et l'éjection du sang dans l'aorte 14. La pulsation du coeur étant telle que la systole possède une durée sensiblement plus faible que celle du cycle cardiaque, la vitesse d'éjection systolique en est d'autant plus élevée. L'élasticité des artères évite de demander corrélativement une puissance excessive au ventricule pour vaincre la charge opposée par l'inertie et la résistance de la colonne sanguine qu'il doit mettre en mouvement et permet ainsi, en quelque sorte, de délivrer l'énergie à débit instantané plus faible pendant la diastole.

L'insuffisance cardiaque est caractérisée par l'incapacité du ventricule à délivrer la quantité de sang nécessaire à l'organisme faute de puissance, qu'il y ait ou non durcissement des artères (mais le durcissement des artères aggrave singulièrement la pathologie).

Dans ce cas, la pompe d'assistance cardiaque joue un double rôle : en premier lieu, elle apporte artificiellement une élasticité complémentaire à l'aorte, comme indiqué ci-dessus, avec le même bénéfice que celui procuré par l'élasticité naturelle du réseau artériel ; en second lieu et selon un aspect original - la pompe a la possibilité de fournir un apport d'énergie supplémentaire au système circulatoire pendant la diastole.

Si l'on considère la représentation modélisée de la figure 2, le volume total produit artificiellement par le déplacement du piston 25 de la pompe d'assistance cardiaque, en fin de déplacement, est donné par :

$$\Sigma . \int_S u(t)\ dt,$$

$\Sigma$ étant la surface du piston et $u(t)$ étant la vitesse, variable en fonction du temps, de déplacement du piston pendant tout ou partie de la systole S.

Ce volume de sang, emmagasiné dans la pompe pendant la systole, est ensuite réinjecté dans l'aorte pendant la diastole D qui succède à la systole, par mouvement inverse du piston à la vitesse $v(t)$, également variable en fonction du temps et pendant tout ou partie de la diastole D (cette vitesse $v(t)$ étant indépendante de $u(t)$).

Du point de vue énergétique, la pompe prélève pendant la systole une énergie $E_i$ :

$$E_1 = \Sigma . \int_S P(t).u(t)\ dt,$$

$P(t)$ étant la pression exercée par le sang sur le piston.

Pendant la diastole, la pompe restitue l'énergie $E_o$ :

$$E_o = \Sigma . \int_D P(t).v(t)\ dt.$$

Le bilan énergétique $E_o - E_i$ du fait de la prothèse peut être :

— négatif, ce qui signifie que le coeur fournit de l'énergie à la prothèse (ce cas est bien évidemment sans intérêt) ;

— nul ou faiblement positif : la pompe se comporte alors essentiellement comme une élastance (facteur d'élasticité) additionnelle, venant s'ajouter à celle, naturelle, de l'artère selon le schéma de fonctionnement exposé plus haut et avec le même bénéfice. Dans ce cas, le coeur subvient seul à la demande énergétique. Ceci peut par exemple s'appliquer à un patient au repos.

— positif : dans ce cas, l'assistance cardiaque est poussée plus loin, l'accroissement substantiel du volume de sang emmagasiné par la pompe et la diminution corrélative de la pression systolique permettent d'augmenter sensiblement le volume d'éjection systolique moyennant une énergie cardiaque inférieure ou égale à ce qu'elle était avant cet accroissement. Outre son rôle d'élastance additionnelle, la pompe fournit pendant la diastole le supplément d'énergie nécessaire pour compenser le déficit du coeur.

En modulant ainsi le volume emmagasiné et l'énergie délivrée, la pompe permet de doser l'assistance cardiaque, cycle à cycle, en fonction de la demande hémodynamique. Le dosage peut notamment être conduit de façon à charger le coeur au maximum de ses capacités opérationnelles en toutes circonstances, ou à une partie de celles-ci au choix du médecin, au moyen de réglages et d'automatismes tels qu'exposés plus bas.

L'avantage majeur d'un dosage proportionnel de ce type est de réduire au maximum la consommation d'énergie du dispositif.

Il est aussi de limiter l'intervention de la machine dans le système circulatoire, afin de perturber le moins possible l'équilibre homéostatique naturel et d'éviter ainsi de graves retentissements à long terme sur les grandes fonctions organiques, comme les fonctions rénales et hépatiques.

On peut enfin espérer que ce mode d'assistance adaptée et limitée favorise la réhabilitation du coeur.

Une telle assistance cardiaque est mise en oeuvre par le circuit de l'invention, qui opère de la manière suivante.

*Données*

Tout d'abord, l'état du système cardiovasculaire est caractérisé par des données accessibles au dispositif par l'intermédiaire de capteurs. La liste des données est la suivante :

- pression aortique en fonction du temps P(t)
- concentration en oxygène veineux $PO_{2V}$
- concentration en oxygène aortique $PO_{2A}$
- fréquence cardiaque F
- contractilité du myocarde dP/dt

Cette liste n'est pas limitative : ainsi, la ventilation-minute MV et/ou la posture et/ou d'autres paramètres encore peuvent également être pris en compte à partir de capteurs appropriés correspondants (par exemple à partir d'accéléromètres pour la posture et l'activité G).

*Acquisition des données*

Les données sont obtenues ainsi :

- P(t) : par un capteur spécifique disposé dans la pompe côté sang ou côté fluide hydraulique.
- $PO_{2V}$ et $PO_{2A}$ : par des capteurs spécifiques disposés l'un 34 dans le coeur droit ($PO_{2V}$), ou ailleurs dans les veines, et l'autre 36 dans le flux aortique traversant la pompe ($PO_{2A}$). Le paramètre utile est $PO_{2A}$ - $PO_{2V}$. L'utilisation de $PO_{2V}$ seulement est envisageable pour faire fonctionner le système, mais moyennant une réduction de performance.
- F : par l'intermédiaire de l'électrocardiogramme (ECG) ou directement à partir du synthétiseur de fréquence de suppléance, le cas échéant. L'ECG est capté par une électrode endocavitaire ventriculaire 38 dans le coeur droit. Il détermine l'instant de la dépolarisation ventriculaire droite, dont on peut déduire l'instant de la dépolarisation du ventricule gauche, et la fréquence cardiaque F. En variante, l'ECG peut être capté par une électrode épicardique placée sur le ventricule gauche.
- dP/dt : ce paramètre est la pente moyenne de la pression dans le ventricule gauche au cours de la contraction isovolumétrique de celui-ci, pente obtenue à partir de l'écart de pression et de la durée entre le début de la dépolarisation et l'ouverture de la valve aortique, détectée par la discontinuité correspondante de P(t).

Après validation, les données sont déduites des mesures effectuées suivant l'usage auquel elles sont destinées :

a) au cours du cycle cardiaque précédent pour un suivi rapide ou une estimation de la tendance, ou
b) à partir de la moyenne pondérée glissante sur un nombre déterminé de cycles précédents, afin d'effectuer un lissage et/ou de permettre aux effets de se stabiliser.

La pression aortique P(t) est échantillonnée à intervalles prédéterminés.

Les données sont variables en fonction de paramètres d'état comme l'activité physique, gastro-intestinale, cérébrale, la posture, l'environnement ou résultant de pathologies coexistantes, de l'imprégnation de drogues, etc. Elles sont aussi dans une certaine mesure interdépendantes.

*Expression du besoin d'assistance cardiaque*

Le besoin d'assistance cardiaque est déterminé à partir d'un ensemble de données et de combinaisons de données de référence. Cet ensemble est établi compte tenu de la pathologie cardiovasculaire et introduit dans le dispositif par programmation.

L'ensemble de référence possède sa propre dynamique en fonction des paramètres d'état : par exemple, l'expression de la normalité à l'effort est traduite par des valeurs de P(t), $PO_{2A}$-$PO_{2V}$, dP/dt différentes de celles qui expriment la normalité au repos. Il en est de même pour les normalités avec ou sans imprégnation pharmaceutique, etc.

La programmation de l'ensemble de référence doit donc être effectuée à deux niveaux :

- celui des données de référence au repos, et
- celui des algorithmes de correction de ces dernières en fonction des variations résultant de l'activité quotidienne. L'activité physique étant à cet égard le facteur essentiel de variation, les algorithmes pourront généralement être limités à la traduction de l'activité physique par l'intermédiaire de la fréquence cardiaque.

L'ensemble de référence sera finalement déterminé par les données à deux variables : P (t,F), $PO_{2A}$(F)-$PO_{2V}$(F), F, dP/dt(F) et/ou la combinaison de ces données.

Dans le cas où l'imprégnation pharmaceutique ou l'état pathologique seraient modifiés, il y aurait lieu de reprendre la programmation à ses deux niveaux.

Une fois la programmation effectuée, les écarts constatés au cours de la vie quotidienne par rapport aux valeurs de référence, passé un certain seuil, déterminent le besoin d'assistance cardiaque. Par exemple l'accroissement de $PO_{2A}$-$PO_{2V}$, ou la chute de $PO_{2V}$, à l'effort déclenchera l'assistance cardiaque alors qu'elle n'était pas requise au repos, ou bien l'était à un moindre degré.

*Assistance cardiaque*

Les modalités de l'assistance sont définies par programmation en fonction de la nature, de l'amplitude et de la vitesse de variation des écarts.

Dès lors qu'une assistance est mise en oeuvre, un programme de suivi est lancé en complément du précédent, comprenant par exemple le renforcement de l'échantillonnage, les algorithmes de démarrage, de poursuite, de terminaison, etc., et réagissant sur la conduite de l'assistance en fonction de l'évolution de la perturbation qui en est la cause et de la réponse de l'organisme.

*Modalités de l'assistance cardiaque*

L'assistance cardiaque est déterminée par les vitesses de déplacement du piston $u(t)$ pendant la systole et $v(t)$ pendant la diastole, respectivement homologues des volumes $\Sigma.u(t)$ accumulé et $\Sigma.v(t)$ restitué par la pompe et de l'instant origine de ces fonctions par rapport au cycle cardiaque, $t_A$ pour $u(t)$ et $t_R$ pour $v(t)$.

Détermination de $u(t)$ et $t_A$ :

L'augmentation artificielle du volume aortique $\Sigma.u(t)$ provoque, comme exposé plus haut, une augmentation du débit systolique qui retentit sur l'énergie délivrée par le ventricule gauche et sur le rendement énergétique de celui-ci. Les effets en sont aussi bénéfiques que ceux de l'assouplissement des artères. Il est en outre important qu'$u(t)$ et $t_A$ soient correctement adaptés à la fonction cardiaque.

Une forme préférentielle du profil de $u(t)$ peut, par exemple, être une décroissance exponentielle, par analogie avec le comportement élastique des artères, tandis que $t_A$ peut contrôler le début de la systole par anticipation afin de réduire la pression systolique.

En pratique, le profil de $u(t)$ et $t_A$ sont réglés en fonction du patient par programmation et ne varient pas en fonction de son activité. L'amplitude de $u(t)$, et par conséquent $\int_S u(t)\, dt$, constitue le paramètre d'action essentiel de l'assistance cardiaque pour ce qui concerne l'augmentation artificielle du volume aortique ou, autrement dit, la diminution de la postcharge du ventricule gauche.

L'amplitude de $u(t)$, en particulier la quantité $\int_S u(t)\, dt$ homologue du volume total emmagasiné par la pompe $\Sigma.\int_S u(t)\, dt$, est déterminée en fonction du débit cardiaque souhaité. Compte tenu du temps de réponse du système cardiovasculaire à l'impulsion qui lui est donnée, l'amplitude $u(t)$ peut être incrémentée progressivement, chaque incrément étant défini en fonction du résultat du précédent dans un délai déterminé, et limité par la capacité de réponse du coeur et de la prothèse.

Détermination de $v(t)$ et $t_R$ :

La réinjection est régie par le profil de $v(t)$ et par $t_R$, l'amplitude de $v(t)$ étant dépendante de celle de $u(t)$ puisque $\int_S u(t)\, dt = \int_D v(t)\, dt$, par le fait que le piston revient à la même position après l'accomplissement d'un cycle.

$v(t)$ et $t_R$ déterminent, à partir de la pression artérielle en début de diastole et en coopération avec la compliance artérielle, la pression artérielle et le débit artériel instantanés pendant la diastole. À l'équilibre, le débit artériel total pendant la diastole, ajouté au débit artériel pendant la systole, est naturellement égal au débit du ventricule gauche.

L'état du patient étant stable, l'introduction d'une assistance cardiaque liée à des valeurs déterminées $u(t)$, $t_A$, $v(t)$, $t_R$ entraîne donc une perturbation suivie d'un rééquilibrage spontané du système cardiovasculaire pour aboutir à un nouveau régime de fonctionnement défini par le débit cardiaque, les diverses pressions ventriculaires et aortiques, systoliques et diastoliques et, par suite, les énergies délivrées par le coeur et par la prothèse.

On comprend donc que :

— si l'assistance cardiaque améliore l'état cardiovasculaire, ses effets exacts ne sont connus qu'après stabilisation de celui-ci, à la lecture de l'ensemble de données ;

— l'assistance cardiaque doit être appliquée de préférence par approches successives,

— l'assistance cardiaque peut être effectuée à différents degrés d'intensité suivant le dosage de la diminution de la postcharge et de l'apport d'énergie de la prothèse.

*Protocole de programmation*

L'assistance cardiaque peut être au choix prédéterminée ou automatique.

L'assistance prédéterminée est obtenue par programmation au repos de $u(t)$, $t_A$, $v(t)$, $t_R$ et de l'algorithme de correction de $u(t)$ et du profil $v(t)$ ainsi que de $t_R$ en fonction de la fréquence cardiaque F.

L'assistance automatique est obtenue par programmation de l'ensemble de référence, des algorithmes de correction de cet ensemble en fonction des paramètres d'état, qui se réduiront en pratique à la fréquence cardiaque F, des seuils de déclenchement de l'assistance et de l'intensité de celle-ci.

La programmation comprend les opérations suivantes :

A. En assistance prédéterminée :

1. Mise en oeuvre des capteurs ; relevé des résultats.

2. Programmation de l'assistance : $u(t)$, $t_A$, $v(t)$,

$t_R$.

3. Programmation des algorithmes de correction de $u(t)$, du profil de $v(t)$ et de $t_R$ en fonction de la fréquence cardiaque.

B. En assistance automatique :

1. Mise en oeuvre des capteurs ; relevé des résultats.
2. Programmation de l'ensemble de référence et des algorithmes de correction.
3. Programmation de la mise en oeuvre de l'assistance en fonction des écarts (seuil et intensité) par rapport à l'ensemble de référence.

*Fonctionnement*

L'appareil programmé effectue les opérations suivantes :

1. Relevé des capteurs, élaboration de l'ensemble des données en temps réel.
2. Correction de l'ensemble de référence en temps réel (assistance automatique).
3. Élaboration des écarts entre l'ensemble des données (1) et l'ensemble de référence (2) (assistance automatique).
4. Déclenchement de l'assistance en fonction des écarts (assistance automatique) ou correction de l'assistance en fonction de la fréquence cardiaque (assistance prédéterminée).
5. Déclenchement du programme de suivi. Protocole d'approches successives (assistance automatique).
6. Enregistrement Holter des données et événements significatifs, des paramètres de fonctionnement, notamment la consommation.
7. Enregistrement de données statistiques.

On notera que l'appareil peut indifféremment corriger l'ensemble de référence (opération 2) ou l'ensemble de données en temps réel (correction inverse).

*Exemple comparatif d'onde de pression obtenue*

La figure 3 illustre la forme de l'onde de pression, c'est-à-dire la fonction P(t), pression aortique en fonction du temps.
La pression est donnée en millimètres de mercure, qui est l'unité hors système dans laquelle les pressions sanguines sont universellement exprimées dans la pratique (1 mm Hg = 133,322 Pa).
La courbe en trait plein représente la pression aortique sans assistance ventriculaire, tandis que la courbe en tiretés représente cette même pression avec assistance ventriculaire, pour une réinjection (diminution du volume variable 20) opérée entre t = 200 ms (fin de systole) et t = 340 ms.

Si l'on considère tout d'abord la courbe de pression sans assistance cardiaque, à l'équilibre la pression en fin de diastole est égale à la pression en début de systole, le cycle étant stabilisé et répétitif.
L'aire délimitée par la courbe P(t) entre t = 0 et t = 750 ms (un cycle complet pour un rythme de 80 pulsations par minute) et l'axe horizontal (arrêté en ordonnées à P = 20 mm Hg, pression en fin de diastole du ventricule gauche) est représentative du débit capillaire total, égal au débit total du coeur :

$$Q_{cardiaque} = \text{aire } (P) / R = 1/R \int_o^{750} P(t)dt,$$

R étant la résistance des capillaires artérioveineux, qui intervient dans la relation $Q_c = P/R$ donnant le débit capillaire.
Si l'on considère maintenant la courbe avec assistance cardiaque (courbe en tiretés), on peut formuler plusieurs observations.
Tout d'abord, bien que la réinjection soit, dans l'exemple illustré, effectuée au début de la diastole, elle pourrait également débuter peu avant la fin de la systole, en prenant toujours fin à t = 340 ms. Après la fin de la réinjection, l'aorte, qui a été dilatée par la réinjection, commence à se décharger de façon approximativement exponentielle dans le réseau artériel aval. De ce fait, la pression diastolique en fin de diastole sera supérieure à cette même pression à la fin de la diastole précédente. Au moment de la mise en service de l'assistance, le cycle ne sera pas équilibré, et il ne le deviendra que quelques cycles plus tard.
En second lieu, on notera que la différence entre la courbe en trait plein et la courbe en tiretés est représentative des différences de débit capillaire avec et sans assistance.
Avec assistance, ce débit capillaire est inférieur pendant la systole et supérieur pendant la diastole. Le bilan (différence entre les aires délimitées par les courbes avant et après l'instant de la réinjection) représente l'apport dû à l'asservissement du débit cardiaque. Le gain exact de débit cardiaque pourra être déterminé après stabilisation du profil de pression, pour tenir compte du fait que la mise en service de l'assistance va modifier un certain nombre de grandeurs physiologiques telles que pressions diastolique et systolique, remplissage du ventricule gauche, énergie du ventricule gauche, résistance R (qui diminue), fréquence F (qui diminue), etc. avec en particulier une meilleure irrigation des coronaires. La stabilisation au nouvel état d'équilibre ne sera donc pas immédiate.
On notera également que le débit cardiaque, représenté par l'aire délimitée par la courbe pendant la durée du cycle, est délivré par le ventricule gauche pendant la systole. C'est-à-dire que l'aire relative à la systole représente le débit capillaire pendant la systole, tandis que le débit cardiaque emmagasiné par là dilatation de l'artère pendant la systole est représenté par l'aire relative à la diastole.

Le profil de pression renseigne ainsi immédiatement sur la part de l'éjection systolique qui est emmagasinée dans l'artère - et dans le volume variable de la pompe si celle-ci est activée - et la part qui est perfusée directement dans le réseau artériel aval (ou dans les capillaires, à la dilatation près des artères en aval).

**Revendications**

1. Un dispositif intraaortique d'assistance cardiaque implantable du type à ballonnet de contrepression, du type comprenant :

   - une pompe (10) comprenant un corps (12) rigide apte à être inséré dans une artère, notamment l'artère aorte descendante, définissant un premier volume (20) traversé par le sang à cet endroit et pourvu d'une membrane souple (16) définissant entre corps et membrane un second volume (18) séparé du premier volume par la membrane,
   - des moyens à volume variable (24) aptes à modifier cycliquement et de manière contrôlée ledit second volume (18) ; et
   - un circuit (26) de pilotage de ces moyens à volume variable (24), dispositif **caractérisé en ce que** le circuit de pilotage (26) comporte des moyens de recueil d'une donnée représentative de l'état du système cardiovasculaire, comportant au moins certains des moyens du groupe comprenant :

     . des moyens pour recueillir une donnée représentative de la pression aortique (P(t)) ;
     . des moyens pour recueillir une donnée représentative de la concentration en oxygène veineux ($PO_{2V}$) ;
     . des moyens pour recueillir une donnée représentative de la concentration en oxygène aortique ($PO_{2A}$) ;
     . des moyens pour recueillir une donnée représentative de la fréquence cardiaque (F) ;
     . des moyens pour recueillir une donnée représentative de la contractilité du myocarde (dP/dt).

2. Le dispositif de la revendication 1, dans lequel le circuit de pilotage comporte des moyens de commande d'assistance cardiaque, comportant au moins certains des moyens du groupe comprenant :

   . des moyens pour piloter la vitesse de variation (u(t)) dudit volume pendant la phase systolique du myocarde ;

   . des moyens pour commander l'instant ($t_A$) de début de cette variation du volume pendant la phase systolique du myocarde ;
   . des moyens pour piloter la vitesse de variation (v(t)) dudit volume pendant la phase diastolique du myocarde ; et des moyens pour commander l'instant ($t_R$) de début de cette variation du volume pendant la phase diastolique du myocarde.

3. Le dispositif de la revendication 2, dans lequel, les moyens de recueil comportant des moyens pour recueillir une donnée représentative de la fréquence cardiaque (F), les moyens de commande d'assistance cardiaque sont des moyens d'assistance prédéterminée, opérant/à partir :

   (i) d'un ensemble de valeurs programmées au repos de paramètres de référence, ces paramètres comprenant au moins l'un des paramètres parmi : la vitesse de variation (u(t)) du volume pendant la phase systolique du myocarde ; la vitesse de variation (v(t)) du volume pendant la phase diastolique du myocarde ; l'instant ($t_A$) de début de ladite variation du volume pendant la phase systolique ; et l'instant ($t_R$) de début de ladite variation du volume pendant la phase diastolique, et
   (ü) d'un algorithme de correction d'au moins l'un desdits paramètres en fonction de la fréquence cardiaque (F).

4. Le dispositif de la revendication 2, dans lequel le circuit de pilotage comporte en outre des moyens pour recueillir une donnée (MV) représentative des besoins métaboliques du patient.

5. Le dispositif de la revendication 2, dans lequel le circuit de pilotage comporte en outre des moyens pour recueillir une donnée (G) représentative de l'activité physique du patient.

6. Le dispositif de la revendication 2, dans lequel les moyens de commande d'assistance cardiaque sont des moyens d'assistance automatique, opérant à partir :

   (i) d'un ensemble, élaboré en temps réel, de données représentatives recueillies comprenant au moins : la pression aortique (P(t)) ; la concentration en oxygène veineux ($PO_{2V}$) ; la concentration en oxygène aortique ($PO_{2A}$) ; la fréquence cardiaque (F) ; et la contractilité du myocarde (dP/dt) ;
   (ü) d'un ensemble de paramètres homologues de référence ;
   (iü) des écarts déterminés entre ledit ensemble de données représentatives et ledit ensemble de référence, et

(iv) d'un algorithme de mise en oeuvre des moyens à volume variable en fonction des écarts ainsi déterminés.

7. Le dispositif de la revendication 6, dans lequel l'algorithme de mise en oeuvre des moyens à volume variable est un algorithme apte à déclencher un programme de suivi de l'assistance cardiaque, notamment par application de l'assistance par approches successives.

8. Le dispositif de la revendication 7, en combinaison avec l'une des revendications 4 ou 5, dans lequel ledit programme de suivi de l'assistance cardiaque est un programme apte à opérer une correction à l'ensemble de données élaboré en temps réel ou à l'ensemble de référence.

**Patentansprüche**

1. Implantierbare Vorrichtung zur Herzunterstützung in der Aorta vom Gegendruckballontyp, von dem Typ, der umfasst:

 - eine Pumpe (10), die einen starren Körper (12) umfasst, der dazu geeignet ist, in eine Arterie eingeführt zu werden, insbesondere die absteigende Aorta, welche ein erstes Volumen (20) definiert, das durch das Blut an dieser Stelle durchquert wird und mit einer nachgiebigen Membran (16) versehen ist, welche zwischen Körper und Membran ein zweites Volumen (18) definiert, das durch die Membran vom ersten Volumen getrennt ist,
 - Mittel mit variablem Volumen (24), dazu geeignet, das zweite Volumen (18) zyklisch und in kontrollierter Weise zu modifizieren; und
 - eine Schaltung (26) zur Steuerung dieser Mittel mit variablem Volumen (24), wobei die Vorrichtung **dadurch gekennzeichnet ist, dass** die Steuerschaltung (26) Mittel zum Empfangen eines repräsentativen Datums des Zustands des kardiovaskulären Systems umfasst, umfassend mindestens bestimmte Mittel aus der Gruppe, die beinhaltet:

 - Mittel zum Empfangen eines Datums, das repräsentativ für den Druck in der Aorta ($P(t)$) ist;
 - Mittel zum Empfangen eines Datums, das repräsentativ für die Konzentration des Sauerstoffs in der Vene ($PO_{2V}$) ist;
 - Mittel zum Empfangen eines Datums, das repräsentativ für die Konzentration von Sauerstoff in der Aorta ($PO_{2A}$) ist;
 - Mittel zum Empfangen eines Datums, das repräsentativ für die Herzfrequenz ($F$) ist; und

 - Mittel zum Empfangen eines Datums, das repräsentativ für die Zusammenziehbarkeit des Herzmuskels ($dP/dt$) ist.

2. Vorrichtung nach Anspruch 1, in welcher die Steuerschaltung Mittel zur Steuerung der Herzunterstützung umfasst, umfassend mindestens bestimmte Mittel aus der Gruppe, die umfasst:

 - Mittel zum Steuern der Änderungsgeschwindigkeit ($u(t)$) des Volumens während der systolischen Phase des Herzmuskels,
 - Mittel zum Steuern des Zeitpunkts ($t_A$) des Beginns dieser Änderung des Volumens während der systolischen Phase des Herzmuskels;
 - Mittel zum Steuern der Änderungsgeschwindigkeit ($v(t)$) des Volumens während der diastolischen Phase des Herzmuskels; und
 - Mittel zum Steuern des Zeitpunkts ($t_A$) des Beginns dieser Änderung des Volumens während der diastolischen Phase des Herzmuskels.

3. Vorrichtung nach Anspruch 2, in welcher, wobei die Mittel zum Empfangen Mittel zum Empfangen eines Datums aufweisen, das repräsentativ für die Herzfrequenz ($F$) ist, die Mittel zur Steuerung der Herzunterstützung Mittel zur vorherbestimmten Unterstützung sind, die arbeiten, ausgehend von:

 (i) einer Gruppe von im Ruhezustand programmierten Werten von Referenzparametern, wobei diese Parameter mindestens einen der Parameter aus: der Änderungsgeschwindigkeit ($u(t)$) des Volumens während der systolischen Phase des Herzmuskels umfassen; der Änderungsgeschwindigkeit ($v(t)$) des Volumens während der diastolischen Phase des Herzmuskels; des Zeitpunkts ($t_A$) des Beginns der genannten Änderung des Volumens während der systolischen Phase; und des Zeitpunkts ($t_R$) des Beginns der genannten Änderung des Volumens während der diastolischen Phase, und (ii) einem Algorithmus zur Korrektur mindestens eines dieser Parameter abhängig von der Herzfrequenz ($F$).

4. Vorrichtung nach Anspruch 2, in welcher die Steuerungsschaltung ferner Mittel zum Empfangen eines Datums (MV) aufweist, das repräsentativ für die metabolischen Bedürfnisse des Patienten ist.

5. Vorrichtung nach Anspruch 2, in welcher die Steuerungsschaltung ferner Mittel zum Empfangen eines Datums (G) aufweist, das repräsentativ für die physische Aktivität des Patienten ist.

6. Vorrichtung nach Anspruch 2, in welcher die Mittel zur Steuerung der Herzunterstützung Mittel zur automatischen Unterstützung sind, arbeitend ausgehend von:

(i) einer Gruppe, in Echtzeit verarbeitet, von empfangenen repräsentativen Daten, die mindestens umfassen: den Druck in der Aorta (P(t)); die Sauerstoffkonzentration in der Vene ($PO_{2v}$); die Sauerstoffkonzentration in der Aorta ($PO_{2A}$); die Herzfrequenz (F) und die Zusammenziehbarkeit des Herzmuskels (dP/dt);
(ii) einer Gruppe von homologen Referenzparametern,
(iii) bestimmten Abständen zwischen der Gruppe von repräsentativen Daten und der Referenzgruppe, und
(iv) einem Algorithmus zur Verwirklichung der Mittel variablen Volumens abhängig von den so bestimmten Abständen.

7. Vorrichtung nach Anspruch 6, in welcher der Algorithmus zur Verwirklichung der Mittel mit variablem Volumen ein Algorithmus ist, der im Stande ist, ein Folgeprogramm der Herzunterstützung auszulösen, insbesondere durch Anwendung der Unterstützung durch aufeinander folgende Annäherungen.

8. Vorrichtung nach Anspruch 7, in Kombination mit einem der Ansprüche 4 oder 5, in welchem das Folgeprogramm zur Herzunterstützung ein Programm ist, das im Stande ist, eine Korrektur der Gruppe der in Echtzeit verarbeiteten Werte oder der Referenzgruppe auszuführen.

**Claims**

1. An implantable intra-aortic heart-assist device of the back-pressure balloon type, of the type comprising:

   - a pump (10) including a rigid body (12) adapted to be inserted in an artery, in particular the down aorta artery, defining a first volume (20) through which blood flows there, and provided with a flexible membrane (16) defining between the body and the membrane a second volume (18) separated from the first volume by the membrane ;
   - variable volume means (24) adapted to cyclically and in controlled manner modify said second volume (18); and
   - a circuit (26) for driving said variable volume means (24), said device being **characterized in that** the driving circuit (26) includes means for sensing data representative of the state of the cardiovascular system, including at least some of the means from the group comprising:

     · means for sensing data representative of aortic pressure (P(t));
     · means for sensing data representative of venous oxygen concentration ($PO_{2V}$);
     · means for sensing data representative of aortic oxygen concentration ($PO_{2A}$):
     · means for sensing data representative of heartbeat frequency (F); and
     . means for sensing data representative of the myocardial contractility (dP/dt).

2. The device of claim 1, wherein the driving circuit includes heart-assist control means including at least some of the means from the group comprising:

   . means for controlling the rate of variation (u(t)) of said volume during the systolic phase of the myocardium;
   . means for controlling the instant ($t_A$) at which said volume variation starts during the systolic phase of the myocardium;
   . means for controlling the rate of variation (v(t)) of said volume during the diastolic phase of the myocardium; and
   . means for controlling the instant ($t_R$) at which said volume variation starts during the diastolic phase of the myocardium.

3. The device of claim 2, wherein, the sensing means including means for sensing data representative of heartbeat frequency (F), the heart-assist control means are means for predetermined assistance, operating on the basis of:

   i) a programmed set of rest values for reference parameters, said parameters comprising at least one of the parameters taken from: the rate (u(t)) at which the volume varies during the systolic phase of the myocardium; the rate (v(t)) at which the volume varies during the diastolic phase of the myocardium; the instant ($t_A$) at which said volume variation during the systolic phase begins; and the instant ($t_R$) at which said volume variation during the diastolic phase begins; and
   ii) an algorithm for correcting at least one of said parameters as a function of the heartbeat frequency (F).

4. The device of claim 2, wherein the driving circuit further includes means for sensing data (MV) representative of the metabolic needs of the patient.

5. The device of claim 2, wherein the driving circuit further includes means for sensing data (G) representative of the physical activity of the patient.

6. The device of claim 2, wherein the heart-assist control means are means for automatic assistance, operating on the basis of:

> i) a sensed representative data set generated in real time comprising at least: the aortic pressure (P(t)); the venous oxygen concentration ($PO_{2V}$); the aortic oxygen concentration ($PO_{2A}$); the heartbeat frequency (F); and the myocardial contractility (dP/dt);
> ii) a corresponding set of reference parameters;
> iii) differences determined between said representative data set and said reference set; and
> iv) an algorithm for implementing the variable volume means as a function of the differences determined in this way.

7. The device of claim 6, wherein the algorithm for implementing the variable volume means is an algorithm adapted to start a heart assistance tracking program, in particular by applying the assistance in successive approximations.

8. The device of claim 7, in combination with any of claims 4 or 5, wherein the heart assistance tracking program is a program adapted to apply a correction to the data set generated in real time, or to the reference set.

FIG_1

FIG_2

FIG_3